# EUROPEAN PATENT SPECIFICATION

(11) **EP 3 870 568 B1**
(45) Date of publication and mention of the grant of the patent: **28.06.2023**
(21) Application number: 19790228.1
(22) Date of filing: 25.10.2019
(51) Int. Cl.: C07D 239/48, C07D 239/42, A61K 31/505, A61P 31/18

(54) **PROCESS FOR THE PREPARATION OF RILPIVIRINE**
VERFAHREN ZUR HERSTELLUNG VON RILPIVIRINE
PROCÉDÉ DE PRÉPARATION DE RILPIVIRINE

(30) Priority: 25.10.2018 EP 18202717
(43) Date of publication of application: 01.09.2021
(73) Proprietor: Minakem, 59640 Dunkerque (FR)
(72) Inventor: PEREZ, Marc, 59800 LILLE (FR); PETIT, Laurent, 59800 LILLE (FR)
(74) Representative: Plasseraud IP
(86) International application number: PCT/EP2019/079267
(87) International publication number: WO 2020/084142

(56) References cited:
- WO-A2-2005/028479
- WO-A2-2012/143937
- WO-A2-2014/009968
- RUBEN LEENDERS ET AL: "Novel Procedure for the Coupling of Sterically Hindered Electron-Deficient Anilines to the 6-Position of the Purine Core", SYNTHETIC COMMUNICATIONS, vol. 41, no. 21, 1 November 2011 (2011-11-01), pages 3246-3250, XP055536819, PHILADELPHIA, PA; US ISSN: 0039-7911, DOI: 10.1080/00397911.2010.517615

## Description

The present invention relates to a new process of preparation of rilpivirine, or a pharmaceutically acceptable salt thereof. Another aspect of the invention concerns compounds of formulae II and III: 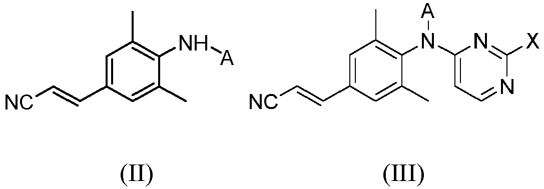 and their salts thereof, and their use for the preparation of Rilpivirine or a suitable salt thereof.

### BACKGROUND OF THE INVENTION

Rilpivirine, chemically 4-{[4-({4-[(E)-2-cyanovinyl]-2,6-dimethylphenyl}amino)-2-pyrimidinyl]amino}benzonitrile, is a drug commonly used for the treatment of HIV infection as an initial therapy in adult patients whose baseline HIV RNA is less than 100 000 copies/mL. It is used as a single 25 mg dose a day, either alone or in combination.

Historically, approaches towards Rilpivirine rely on the addition of (E)-3-(4-amino-3,5-dimethylphenyl)acrylonitrile *i* to 4-((4-chloropyrimidin-2-yl)amino)benzonitrile *ii:*

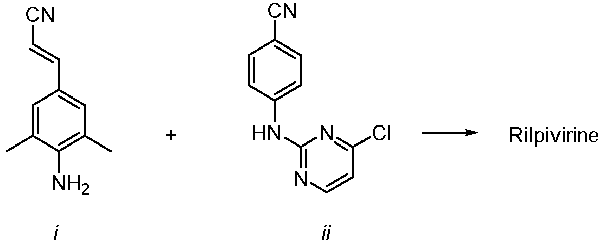

WO 03/016306 discloses the addition of *i* to *ii* at 150°C without any solvent. The same route is used in WO 2004/016581 and in WO 2012/143937 in which the hydrochloride salt of *i* is reacted with *ii* in acetonitrile at reflux. WO 2012/147091 further discloses the reaction of the hydrochloride salt of *i* with *ii* in 1,4-dioxane at 100-110°C in the presence of p-toluene sulfonic acid monohydrate, while the reaction of the hydrochloride salt of *i* with *ii* in the presence of a phase transfer catalyst (e.g. Bu₄N⁺AcO⁻) in a solvent such as acetonitrile is described in WO 2013/179105. Another example of synthesis of Rilpivirine according to this strategy is described in WO 2016/116074 which discloses performing the reaction in methyl isobutyl ketone as solvent in the presence of water.

This reaction requires at least one of the following: prolonged reaction time, high temperature or use of a Class II solvent (as defined within ICH guideline Q3C R6 on impurities). In addition, the synthesis of *ii* requires several chemical steps from 2,4-dichloropyrimidine, the latter being commonly synthesized from uracil and phosphorous (V) oxychloride.

WO 2014/009968 discloses another approach consisting in first reacting the hydrochloride salt of (E)-3-(4-amino-3,5-dimethylphenyl)acrylonitrile *i* with 2,4-dichloropyrimidine to afford (E)-3-(4-(2-chloropyrimidin-4-ylamino)-3,5-dimethylphenyl)acrylonitrile *iii* and then reacting *iii* with 4-aminobenzonitrile to afford Rilpivirine.

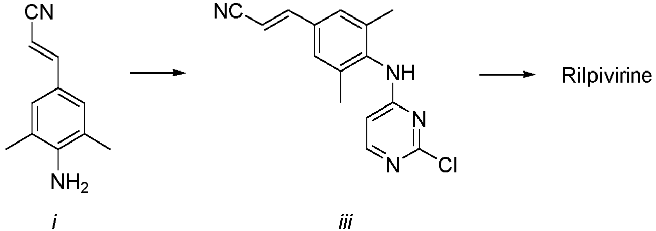

This process provides Rilpivirine in high yields and less steps than the prior art processes. Albeit a Class III solvent (as defined within ICH guideline Q3C R6 on impurities) is used, the formation of the scaffold *iii* from 2,4-dichloropyrimidine and *i* is carried out at high temperature (140-150°C) over a prolonged time (55-60 h). Furthermore, this step is hampered by the use of very large volumes of solvent which render it incompatible with an economical synthesis. Rilpivirine base is obtained in 96.5% purity by HPLC and is purified and converted to the hydrochloride salt using impractical volumes of solvents.

Therefore, there is still a need for a process of preparation of Rilpivirine that requires fewer steps, avoids solvents of concern and proceeds under mild conditions, while being inherently safe and scalable.

### SUMMARY OF THE INVENTION

The inventors have now succeeded in developing a novel process for the preparation of Rilpivirine in good yield that is respectful of the principles of Green Chemistry and that is viable at an industrially relevant scale.

The present invention therefore relates to a process for the preparation of Rilpivirine of formula I, 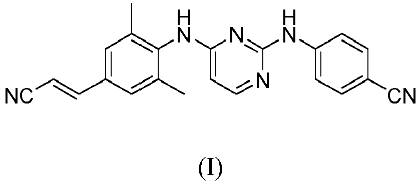 or a pharmaceutically acceptable salt or solvate thereof, comprising the following steps in the following order:
a) preparing a compound of formula II: 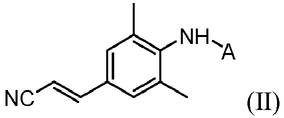 wherein A is an amine protecting group selected from alkyloxycarbonyl, arylalkyloxycarbonyl, alkylcarbonyl, arylsulfonyl, alkylsulfonyl, haloalkylsulfonyl, alkyl, alkenyl and arylalkyl,
b) reacting said compound of formula II with a 2,4-dihalogenopyrimidine in the presence of a base to obtain compound of formula III: 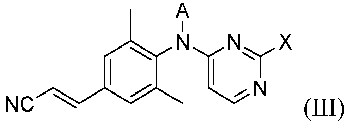 wherein **A** is an amine protecting group selected from alkyloxycarbonyl, arylalkyloxycarbonyl, alkylcarbonyl, arylsulfonyl, alkylsulfonyl, haloalkylsulfonyl, alkyl, alkenyl and arylalkyl and **X** is a halogen,
c) reacting said compound of formula III with 4-aminobenzonitrile in the presence of an acid, and
d) cleaving the group **A**,
wherein steps a), b, and c) are carried out successively in this order and step d) is carried out concomitantly with step c) or after step c).

The invention also relates to a compound of formula II: 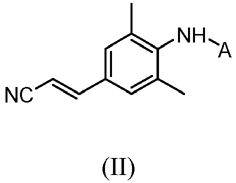 and suitable salts thereof,
wherein A is an amine protecting group selected from alkyloxycarbonyl, alkylcarbonyl, arylsulfonyl, alkylsulfonyl, haloalkylsulfonyl, alkyl, alkenyl and arylalkyl.

The invention also relates to a compound of formula III: 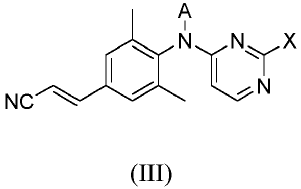 and suitable salts thereof,
wherein **A** is an amine protecting group selected from alkyloxycarbonyl, arylalkyloxycarbonyl, alkylcarbonyl, arylsulfonyl, alkylsulfonyl, haloalkylsulfonyl, alkyl, alkenyl and arylalkyl and **X** is a halogen.

The invention further relates to the use of a compound of formula II as a synthetic intermediate for the preparation of Rilpivirine or a pharmaceutically acceptable salt or solvate thereof, and to the use of a compound of formula III as a synthetic intermediate for the preparation of Rilpivirine or a pharmaceutically acceptable salt or solvate thereof.

### DETAILED DESCRIPTION OF THE INVENTION

The present invention relates to a process for the preparation of Rilpivirine of formula I, 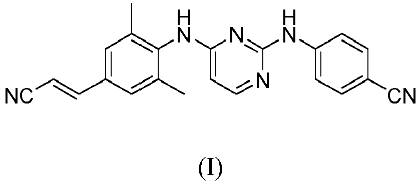 or a pharmaceutically acceptable salt or solvate thereof, comprising the following steps in the following order:
a) preparation of a compound of formula II: 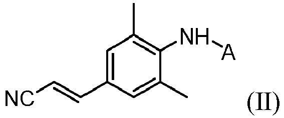 wherein **A** is an amine protecting group selected from alkyloxycarbonyl, arylalkyloxycarbonyl, alkylcarbonyl, arylsulfonyl, alkylsulfonyl, haloalkylsulfonyl, alkyl, alkenyl and arylalkyl, preferably **A** is selected from *tert*-butyloxycarbonyl, *tert*-butylcarbonyl, *p*-toluenesulfonyl, trifluoromethylsulfonyl, *tert*-butyl, allyl, benzyl and trityl, more preferably **A** is *tert*-butyloxycarbonyl,
b) reaction of said compound of formula II with a 2,4-dihalogenopyrimidine, preferably 2,4-dichloropyrimidine, in a presence of a base to obtain compound of formula III: 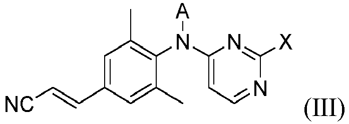 wherein **A** is an amine protecting group selected from alkyloxycarbonyl, arylalkyloxycarbonyl, alkylcarbonyl, arylsulfonyl, alkylsulfonyl, haloalkylsulfonyl, alkyl, alkenyl and arylalkyl, preferably **A** is selected from *tert*-butyloxycarbonyl, *tert*-butylcarbonyl, *p*-toluenesulfonyl, trifluoromethylsulfonyl, *tert*-butyl, allyl, benzyl and trityl, more preferably **A** is *tert*-butyloxycarbonyl, and **X** is a halogen, preferably chloro, and
c) reaction of said compound of formula III with 4-aminobenzonitrile in the presence of an acid with concomitant cleavage of the group **A**, or reaction of said compound of formula III with 4-aminobenzonitrile in the presence of an acid followed by an additional step of cleavage of the group **A**,
d) cleaving group **A**,
wherein steps a), b, and c) are carried out successively in this order and step d) is carried out concomitantly with step c) or after step c).

In a particular embodiment, the process for the preparation of Rilpivirine of formula I, or a pharmaceutically acceptable salt or solvate thereof, comprises the steps a), b), c) and d) as defined above, wherein, in the compound of formula II and the compound of formula III, **A** is a group selected from alkyloxycarbonyl, alkylcarbonyl, arylsulfonyl, alkylsulfonyl, haloalkylsulfonyl, alkyl, alkenyl and arylalkyl. Preferably, **A** is a group selected from alkyloxycarbonyl, arylsulfonyl and alkylsulfonyl. More preferably, **A** is a group selected from alkyloxycarbonyl and alkylsulfonyl. Still more preferably, **A** is a group selected from *tert*-butyloxycarbonyl, methoxycarbonyl, *p*-toluenesulfonyl and methylsulfonyl. Even more preferably, **A** is a group selected from *tert*-butyloxycarbonyl, methoxycarbonyl and methylsulfonyl.

In fact, and without wanting to be tied to any theory whatsoever, the inventors think that the presence of the group **A** in compounds of formula II and III is beneficial in terms of reactivity and selectivity during the course of the reactions involved in the process of the invention, and that the group **A** serves as a chemical handle to drive the reactions involved in the process of the invention.

The step a) of the process of the invention consists in preparing a compound of formula II by reacting (2E)-3-(4-amino-3,5-dimethylphenyl)prop-2-enenitrile with anhydrides, alkanoyl chorides or alkyl chloroformiates, preferably with anhydrides, more preferably with di-*tert*-butyl dicarbonate.

(2E)-3-(4-amino-3,5-dimethylphenyl)prop-2-enenitrile may be obtained by breaking the salt of (2E)-3-(4-amino-3,5-dimethylphenyl)prop-2-enenitrile hydrochloride using the procedure known by the skilled person in the art, such as, for example, suspending (2E)-3-(4-amino-3,5-dimethylphenyl)prop-2-enenitrile hydrochloride in a solvent or a mixture of solvents selected from 2-methyl-tetrahydrofuran, water, toluene, isopropyl acetate, preferably a mixture of 2-methyl-tetrahydrofuran and water, and adding a base selected from sodium hydroxide, sodium hydrogen carbonate and potassium hydrogen carbonate, preferably sodium hydroxide.

Advantageously, the step a) of the process of the invention is performed at reflux in a solvent selected from organic solvents, water, ionic liquids and hexafluoroisopropanol, and mixtures thereof. Preferably, the solvent used in the step a) of the process of the invention is selected from aprotic polar solvents. More preferably, the solvent used in the step a) of the process of the invention is selected from cyclopentyl methyl ether, *tert*-amyl methyl ether, methyl *tert*-butyl ether, tetrahydrofuran and 2-methyl-tetrahydrofuran. Even more preferably, the solvent used in the step a) of the process of the invention is 2-methyl-tetrahydrofuran.

At the end of the reaction, compound of formula II is obtained and used in the next step.

The step b) of the process of the invention consists in reacting compound of formula II with a 2,4-dihalogenopyrimidine, preferably 2,4-dichloropyrimidine, in a presence of a base to obtain compound of formula III.

The base used in the step b) of the process is selected from alkali metal salts of alcohols and amides. Preferably, the base is selected from the group consisting of sodium methoxide, sodium ethoxide, sodium *tert*-butoxide, sodium *tert*-pentoxide, sodium hydroxide, sodium hexamethyldisilazane, potassium methoxide, potassium ethoxide, potassium *tert*-butoxide, potassium *tert*-pentoxide, potassium hydroxide and potassium hexamethyldisilazane. More preferably, the base is selected from the group consisting of sodium *tert*-butoxide, sodium *tert*-pentoxide, sodium hydride, sodium hexamethyldisilazane and potassium *tert*-butoxide. Even more preferably, the base is sodium *tert*-pentoxide.

Advantageously, the step b) of the process of the invention is performed in a solvent selected from polar aprotic solvents and aromatic non-polar solvents. Preferably, the solvent used in the step b) of the process is selected from the group consisting of toluene, xylene, benzene, tetrahydrofuran, 2-methyl-tetrahydrofuran, *N*-methylpyrrolidone, *N,N-*dimethylformamide, acetonitrile, dimethylsulfoxide, *iso*-propyl acetate and ethyl acetate.

More preferably, the solvent is selected from the group consisting of toluene, 2-methyl-tetrahydrofuran, *N*-methylpyrrolidone, *N*,*N*-dimethylformamide, dimethylsulfoxide and *iso*-propyl acetate. Still more preferably, the solvent is selected from the group consisting of *N*-methylpyrrolidone, *N*,*N*-dimethylformamide, and dimethylsulfoxide. Even more preferably, the solvent is dimethylsulfoxide.

Advantageously, the step b) of the process of the invention is run at a temperature ranging from 10°C to 60°C.

Unexpectedly, the inventors found that the reaction conditions used during the step b) of the process of the invention renders the process safe by avoiding the use of sodium hydride in combination with *N*,*N-*dimethylformamide, while being compatible with a use at an industrially relevant scale.

In one embodiment, at the end of the reaction, the reaction mixture may be quenched using the procedures known by the skilled person in the art, such as, for example, adding water with or without the presence of an alcoholic solvent such as iso-propanol or n-butanol, or adding a saturated aqueous solution of ammonium chloride, followed by extraction with an organic solvent, preferably an ethereal solvent, more preferably a solvent selected from methyl *tert*-butyl ether, 2-methyl-tetrahydrofuran, diisopropylether, and cyclopentyl methyl ether.

After the reaction of the step b) of the process of the invention is ended, compound of formula III is obtained and may be purified using the procedures known by the skilled person in the art, such as, for example, recrystallization or purification by column chromatography.

The step c) of the process of the invention consists in reacting compound of formula III obtained in the step b) of the process with 4-aminobenzonitrile in a presence of an acid.

The acid used in the step c) of the process of the invention is selected from acetic acid, hydrochloric acid, *p*-toluenesulfonic acid, methanesulfonic acid, formic acid, trifluoroacetic acid, any supported acidic catalyst (such as clays, Nafion, Amberlyst, and the like) and mixtures thereof. Preferably, the acid used in the step c) of the process is selected from acetic acid, hydrochloric acid, *p*-toluenesulfonic acid, methanesulfonic acid and mixtures thereof. More preferably, the acid used in the step c) of the process of the invention is selected from hydrochloric acid, *p*-toluenesulfonic acid, methanesulfonic acid and mixtures thereof. Even more preferably, the acid used in the step c) of the process of the invention is hydrochloric acid.

Advantageously, the step c) of the process of the invention is performed in a solvent selected from polar solvents and aromatic non-polar solvents, optionally as a mixture with water. Preferably, the solvent used in the step c) of the process is selected from the group consisting of toluene, 2-methyl-tetrahydrofuran, *N*-methyl-pyrrolidone, dimethylsulfoxide, acetonitrile, sulfolane, methanol, ethanol, *n*-propanol, *iso*-propanol, *n*-butanol, 2-methyl-2-butanol, acetone, tetrahydrofuran, chloroform, cyclohexanone, dichloromethane, butan-2-one, dioxane and glacial acetic acid, optionally as a mixture with water. More preferably, the solvent used in the step c) of the process is selected from the group consisting of *N-*methyl-pyrrolidone, acetonitrile, sulfolane, methanol, *n*-propanol, *n*-butanol, 2-methyl-2-butanol, and glacial acetic acid, optionally as a mixture with water. Even more preferably, the solvent used in the step c) of the process is a mixture of glacial acetic acid and water.

Advantageously, the step c) of the process of the invention is performed at a temperature ranging from 50°C to 200°C, more preferably from 50°C to 160°C, even more preferably at 50°C to 120°C.

In one embodiment, the group A is advantageously cleaved under the reaction conditions used in the step c) of the process of the invention between compound of formula III with 4-aminobenzonitrile and Rilpivirine is obtained directly as a salt or solvate, preferably a hydrochloride salt.

In another embodiment, the group **A** is still present at the end of the reaction of the step c) of the process of the invention between compound of formula III with 4-aminobenzonitrile. The process of the invention then comprises an additional step d) of cleavage of the group **A** to obtain Rilpivirine or a pharmaceutically acceptable salt or solvate thereof. This additional step of cleavage of the group **A** may be performed using reactions known by the person skilled in the art for the deprotection of a secondary amine such as, for example those reported in Greene, "Protective Groups in Organic Synthesis," John Wiley and Sons, Fourth Edition (2007).

In a particular embodiment, the process according to the invention is a process for the preparation of Rilpivirine hydrochloride or a solvate thereof. In other terms, the acid in step c) is hydrochloric acid. In a particularly advantageous variant of this embodiment, the group **A** is cleaved concomitantly to the reaction of the compound of formula III with 4-aminobenzonitrile in the presence of hydrochloric acid, i.e. step d) is carried out concomitantly with step c), using a mixture of glacial acetic acid and water as solvent. Under these conditions, the process according to the invention affords polymorphic form A of Rilpivirine hydrochloride. Advantageously, the volume ratio of glacial acetic acid to water is 1:9 to 9:1, preferably 1:1 to 5:1, more preferably about 3:1. Polymorphic form A of Rilpivirine hydrochloride is described in US 7,956,063 B2. It is caracterized by typical X-ray powder diffraction peaks at two-theta positions 9.7°±0.2°, 13.5°±0.2°, 15.0°±0.2°. Form A is further characterized by X-ray powder diffraction peaks at two-theta positions 9.1°±0.2°, 11.0°±0.2°, 14.6°±0.2°, 22.0°±0.2°, 25.0°±0.2°, 25.3°±0.2° and 26.7°±0.2°.

Other polymorphic forms may be obtained when other solvents are used. Particularly, polymorphic form B may be obtained when the solvent is propan-2-one, polymorphic forms C or D may be obtained when the solvent is glacial acetic acid. Polymorphic form E may be obtained when the solvent is ethanol, and polymorphic form F may be obtained when the solvent is methanol, 2-propanol, 1-propanol, acetone, tetrahydrofuran, chloroform, cyclohexanone, dichloromethane, butan-2-one or dioxane.

Rilpivirine or its salts or solvates may be isolated after the step c) of the process or after the additional step of cleavage of the group **A** by the methods known by the person skilled in the art such as filtration or centrifugation, preferably by filtration.

Rilpivirine or its salts or solvates may then be purified using the procedures known by the person skilled in the art, such as, for example, recrystallization or purification by column chromatography.

In one embodiment, Rilpivirine is obtained as a solvate. The nature of the solvate depends on the solvents used during the step c) of the process of the invention.

Another aspect of the invention concerns a compound of formula II: 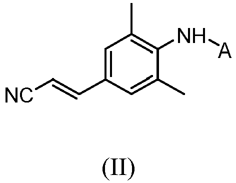 and suitable salts thereof,
wherein
**A** is an amine protecting group selected from alkyloxycarbonyl, alkylcarbonyl, arylsulfonyl, alkylsulfonyl, haloalkylsulfonyl, alkyl, alkenyl and arylalkyl, preferably **A** is group selected from alkyloxycarbonyl, alkylcarbonyl, arylsulfonyl, haloalkylsulfonyl, alkyl, alkenyl and arylalkyl, more preferably **A** is alkyloxycarbonyl.

Preferred compounds of formula II are compounds wherein

**A** is a group selected from *tert*-butyloxycarbonyl, *tert*-butylcarbonyl, *p*-toluenesulfonyl, trifluoromethylsulfonyl, *tert*-butyl, allyl, benzyl and trityl, more preferably **A** is *tert-*butyloxycarbonyl.

In a particular embodiment, compounds of formula II and suitable salts thereof are those wherein **A** is a group selected from alkyloxycarbonyl, alkylcarbonyl, arylsulfonyl, alkylsulfonyl, haloalkylsulfonyl, alkyl, alkenyl and arylalkyl. Preferably, **A** is a group selected from alkyloxycarbonyl, arylsulfonyl and alkylsulfonyl. More preferably, **A** is a group selected from alkyloxycarbonyl and alkylsulfonyl.

According to this embodiment, preferred compounds of formula II are compounds wherein

**A** is a group selected from *tert*-butyloxycarbonyl, methoxycarbonyl, *p*-toluenesulfonyl and methylsulfonyl, more preferably **A** is a group selected from *tert*-butyloxycarbonyl, methoxycarbonyl and methylsulfonyl.

The invention also relates to a compound of formula III: 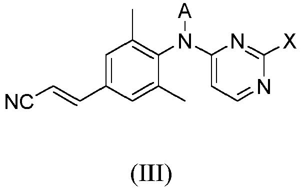 and suitable salts thereof,
wherein
**A** is an amine protecting group selected from alkyloxycarbonyl, arylalkyloxycarbonyl, alkylcarbonyl, arylsulfonyl, alkylsulfonyl, haloalkylsulfonyl, alkyl, alkenyl and arylalkyl, preferably **A** is group selected from alkyloxycarbonyl, alkylcarbonyl, arylsulfonyl, haloalkylsulfonyl, alkyl, alkenyl and arylalkyl, more preferably **A** is alkyloxycarbonyl; and
**X** is a halogen, preferably **X** is chloro.

Preferred compounds of formula III are compounds wherein
**A** is a group selected from tert-butyloxycarbonyl, *tert*-butylcarbonyl, *p*-toluenesulfonyl, trifluoromethylsulfonyl, *tert*-butyl, allyl, benzyl and trityl, more prefereably **A** is *tert-*butyloxycarbonyl; and
**X** is chloro.

In a particular embodiment, compounds of formula III and suitable salts thereof are those wherein **X** is as defined above and **A** is a group selected from alkyloxycarbonyl, alkylcarbonyl, arylsulfonyl, alkylsulfonyl, haloalkylsulfonyl, alkyl, alkenyl and arylalkyl. Preferably, **A** is a group selected from alkyloxycarbonyl, arylsulfonyl and alkylsulfonyl. More preferably, **A** is a group selected from alkyloxycarbonyl and alkylsulfonyl.

According to this embodiment, preferred compounds of formula III are compounds wherein
**A** is a group selected from *tert*-butyloxycarbonyl, methoxycarbonyl, *p*-toluenesulfonyl and methylsulfonyl, more preferably **A** is a group selected from *tert*-butyloxycarbonyl, methoxycarbonyl and methylsulfonyl.

The presence of the group **A** in compounds of formula II and III is beneficial in terms of reactivity and selectivity during the course of the reactions involved in the process of the invention and the group **A** present in compounds of formula II and III is not intended to induce chemoselectivity as would a usual protecting group, but as a chemical handle to drive the reactions involved in the process of the invention. Compounds of formula II and III are thus both key intermediates in the process of the invention.

Another aspect of the invention therefore concerns the use of compound of formula II as a synthetic intermediate for the preparation of Rilpivirine or a pharmaceutically acceptable salt or solvate thereof.

In particular, the compound of formula II is used in the step b) of the process according to the invention consisting in the reaction of said compound of formula II with a 2,4-dihalogenopyrimidine in the presence of a base.

In the same way, the invention also relates to the use of compound of formula III as a synthetic intermediate for the preparation of Rilpivirine or a pharmaceutically acceptable salt or solvate thereof.

In particular, the compound of formula III is used in the step c) of the process according to the invention consisting in the reaction of said compound of formula III with 4-aminobenzonitrile in the presence of an acid.

### DEFINITIONS

The definitions and explanations below are for the terms as used throughout the entire application, including both the specification and the claims.

Unless otherwise stated any reference to compounds of the invention herein, means the compounds as such as well as their pharmaceutically acceptable salts and solvates.

When describing the process and compounds of the invention, the terms used are to be construed in accordance with the following definitions, unless indicated otherwise.

The term "amine protecting group" or "amine protective group" as used herein refers to a chemical group that is introduced into a molecule by chemical modification of a primary or a secondary amino group and, after one or more subsequent chemical reactions, that may then be removed to recover the free primary or secondary amino group. Non-limiting examples of such amine protecting group are provided in Greene, "Protective Groups in Organic Synthesis," John Wiley and Sons, Fourth Edition (2007). Preferred examples of amine protecting groups according to the invention are selected from alkyloxycarbonyl, arylalkyloxycarbonyl, alkylcarbonyl, arylsulfonyl, alkylsulfonyl, haloalkylsulfonyl, alkyl, alkenyl and arylalkyl. More preferred amine protecting groups according to the invention are selected from *tert*-butyloxycarbonyl, *tert*-butylcarbonyl, *p*-toluenesulfonyl, trifluoromethylsulfonyl, *tert*-butyl, allyl, benzyl and trityl. An even more preferred amine protecting group according to the invention is *tert*-butyloxycarbonyl.

The term "alkyl" by itself or as part of another substituent refers to a hydrocarbyl radical of Formula CₙH₂ₙ₊₁ wherein n is an integer greater than or equal to 1.

The term "alkenyl" by itself or as part of another substituent refers to a hydrocarbyl radical formed from an alkene by removal of one hydrogen atom of Formula CₙH₂ₙ₋₁ wherein n is an integer greater than or equal to 2.

The term "halo" or "halogen" means fluoro, chloro, bromo, or iodo. Preferred halo groups are fluoro and chloro, fluoro being particularly preferred.

The term "haloalkyl" alone or in combination, refers to an alkyl radical having the meaning as defined above wherein one or more hydrogens are replaced with a halogen as defined above. Non-limiting examples of such haloalkyl radicals include chloromethyl, 1-bromoethyl, fluoromethyl, difluoromethyl, trifluoromethyl, 1,1,1-trifluoroethyl and the like.

The term "aryl" as used herein refers to a polyunsaturated, aromatic hydrocarbyl group having a single ring (*i.e*. phenyl) or multiple aromatic rings fused together (e.g. naphthyl), typically containing 5 to 12 atoms; preferably 6 to 10, wherein at least one ring is aromatic. Examples of aryl groups include but are not limited to phenyl, naphthyl, anthracyl. Preferred aryl group according to the invention is phenyl.

The term "carbonyl" or "carbonyl group" as used herein refers to a functional group composed of a carbon atom double-bonded to an oxygen atom: C=O.

The term "sulfonyl" as used herein refers to a functional group composed of a sulfur atom double-bonded to two oxygen atoms: O=S=O.

The term "polar aprotic solvents" as used herein refers to solvents having large dipole moments, i.e. molecules having bonds between atoms with very different electronegativities, while being unable to participate in hydrogen bonding.

The term "non-polar solvents" as used herein refers to solvents having dipole moment equal or close to zero, i.e. molecules having no polar group or molecules comprising polar groups but whose geometry causes the dipole moment to vanish.

The compounds of the invention of Formulae II and III containing a basic functional group may be in the form of pharmaceutically acceptable salts. Pharmaceutically acceptable salts of the compounds of the invention containing one or more basic functional groups include in particular the acid addition salts thereof. Suitable acid addition salts are formed from acids which form non-toxic salts. Examples include the acetate, adipate, aspartate, benzoate, besylate, bicarbonate/carbonate, bisulphate/sulphate, borate, camsylate, cinnamate, citrate, cyclamate, edisylate, esylate, formate, fumarate, gluceptate, gluconate, glucuronate, hexafluorophosphate, hibenzate, hydrochloride/chloride, hydrobromide/bromide, hydroiodide/iodide, isethionate, lactate, malate, maleate, malonate, mesylate, methylsulphate, naphthylate, 2-napsylate, nicotinate, nitrate, orotate, oxalate, palmitate, pamoate, phosphate/hydrogen phosphate/dihydrogen phosphate, pyroglutamate, saccharate, stearate, succinate, tannate, tartrate, tosylate, trifluoroacetate and xinofoate salts.

Pharmaceutically acceptable salts of compounds of Formulae II and III and subformulae may for example be prepared as follows:
(i) reacting the compound of Formula I or any of its subformulae with the desired acid; or
(ii) converting one salt of the compound of Formula I or any of its subformulae to another by reaction with an appropriate acid or by means of a suitable ion exchange column.

All these reactions are typically carried out in solution. The salt may precipitate from solution and be collected by filtration or may be recovered by evaporation of the solvent.

The degree of ionization in the salt may vary from completely ionized to almost nonionized.

The term "solvate" is used herein to describe a molecular complex comprising Rilpivirine or a pharmaceutically acceptable salt obtained by the process of the invention and one or more pharmaceutically acceptable solvent molecules. The term 'hydrate' is employed when said solvent is water.

The compounds of the invention include compounds of the invention as hereinbefore defined, including all polymorphs and crystal habits thereof, prodrugs and isomers thereof (including optical, geometric and tautomeric isomers) and isotopically-labeled compounds of the invention.

In addition, although generally, with respect to the salts of the compounds of the invention, pharmaceutically acceptable salts are preferred, it should be noted that the invention in its broadest sense also includes non-pharmaceutically acceptable salts, which may for example be used in the isolation and/or purification of the compounds of the invention.

The present invention will be better understood with reference to the following examples and figures. These examples are intended to be representative of specific embodiments of the invention and are not intended as limiting the scope of the invention.

### FIGURES

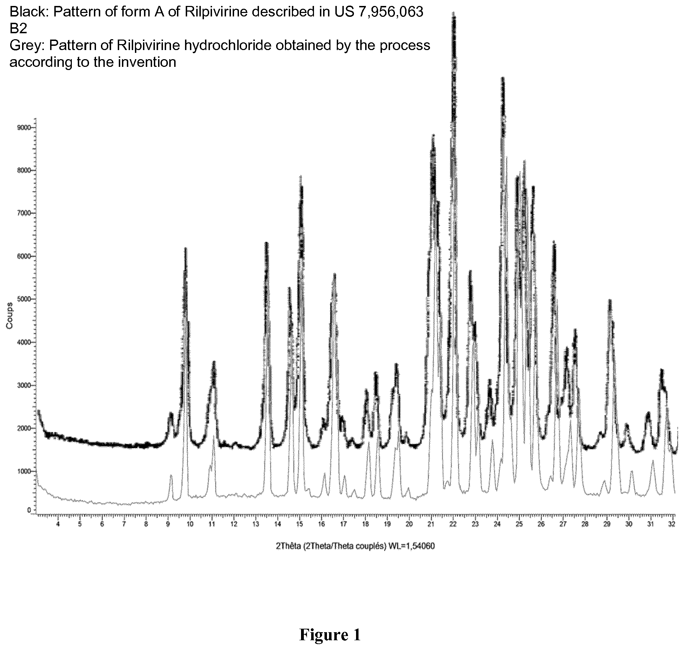

**Figure 1****:** X-ray powder diffraction powder of hydrochloride salt of Rilpivirine (polymorphic form A) obtained by the process according to the invention in comparison with the data of US 7,956,063 B2.

### EXAMPLES

All temperatures are expressed in °C and all reactions were carried out at room temperature (RT) unless otherwise stated.

The reaction monitoring was performed by reverse phase HPLC

NMR spectra were recorded on Bruker Avance 300 (operating at 300 MHz for ¹H and 75 MHz for ¹³C) or Oxford Instrument Pulsar (operating at 60 MHz for ¹H). Chemical shifts are expressed in ppm relative to tetramethylsilane (TMS) or to residual proton signal in deuterated solvents. Chemical shifts are reported as position (*δ* in ppm), multiplicity (s = singulet, d = doublet, t = triplet, q = quartet, m = multiplet, br = broad), coupling constant (*J* in Hz) and relative integral.

The purity of final compounds was determined by high pressure liquid chromatography (HPLC) using Agilent apparatus fitted with a Zorbax SB phenyl column (150 × 6.6 mm, 3.5 µm) using a mixture of H₂O (+0,1% H₃PO₄) and CH₃CN as eluent.

The melting point analyses were performed on Mettler Toledo DSC 3

Solvents, reagents and starting materials were purchased from well-known chemical suppliers (such as for example Sigma Aldrich, Acros Organics, Fluorochem, Eurisotop, VWR International, ABCR, TCI) and the following abbreviations are used:
HPLC: high performance liquid chromatography
DSC: differential scanning calorimetry
m.p.: melting point
LCAP: Liquid Chromatography Area Percentage

### Example 1

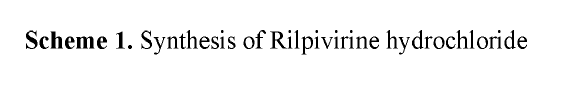

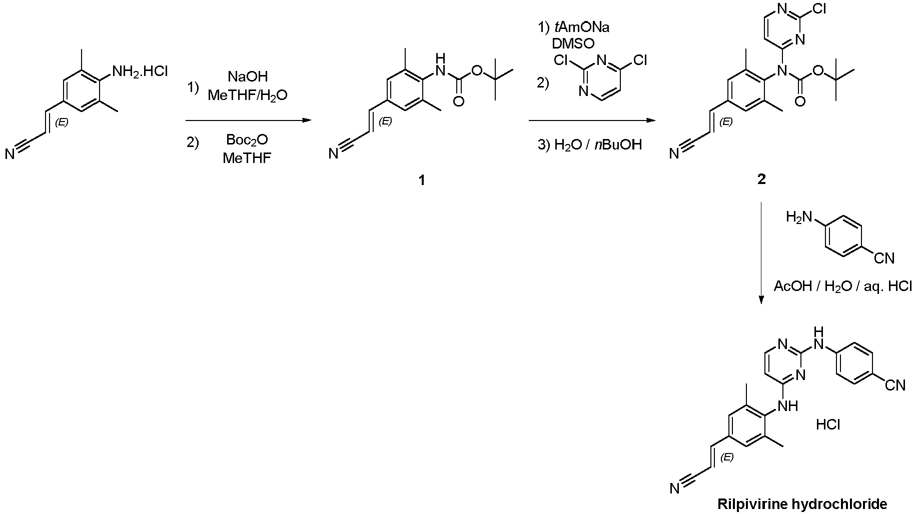

### Salt break of (2E)-3-(4-amino-3,5-dimethylphenyl)prop-2-enenitrile hydrochloride.

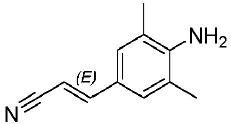

In a round bottomed flask, equipped with a mechanical stirrer, a water condenser and a temperature controller, (2E)-3 -(4-amino-3,5 -dimethylphenyl)prop-2-enenitril e hydrochloride (100 g), 2-methyl-tetrahydrofuran (500 mL) and deionized water (200 mL) are introduced. The mixture is heated at 40°C and a 30% by weight aqueous solution of sodium hydroxide (64 g) is added. After 1 h, the layers are separated, the organic layer is washed with deionized water (50 mL). The solvent is evaporated, the residue (82.7 g, quantitative yield) is diluted with 2-methyl-tetrahydrofuran (150 mL) and is used directly in the next step.

### Synthesis of tert-butyl {4-[(E)-2-cyanoethenyl]-2,6-dimethylphenyl}carbamate 1.

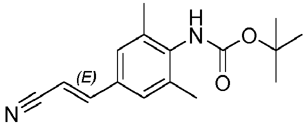

In a round bottomed flask, equipped with a mechanical stirrer, a water condenser, a temperature controller, under inert gas, (2E)-3-(4-amino-3,5-dimethylphenyl)prop-2-enenitrile in 2-methyl-tetrahydrofuran from previous step is heated to reflux. A solution of di-*tert*-butyl dicarbonate in 2-methyl-tetrahydrofuran (299 g of a 70% by weight solution) is added and the resulting mixture is refluxed overnight. After this period of time, 2-methyl-tetrahydrofuran is evaporated, the residue is taken up in dimethylsulfoxide (300 mL). The solvent is evaporated and the residue is taken up in dimethylsulfoxide (300 mL). The solvent is evaporated and the residue is taken up in dimethylsulfoxide (200 mL). The resulting solution containing *tert*-butyl {4-[(E)-2-cyanoethenyl]-2,6-dimethylphenyl}carbamate (118 g, 90% yield) is used directly in the next step. m.p. (DSC) 153 °C.

¹H NMR (60 MHz, CDCl₃): δ (ppm) 7.28 (d, *J* = 10.9 Hz, 1H), 7.08 (s, 2H), 5.85 (s, 1H), 5.71 (d, *J* = 16.5 Hz, 1H), 2.21 (s, 6H), 1.42 (s, 9H). All signals appear broad.

### Synthesis of tert-butyl (2-chloropyrimidin-4-yl){4-[(E)-2-cyanoethenyl]-2,6-dimethylphenyl}carbamate 2.

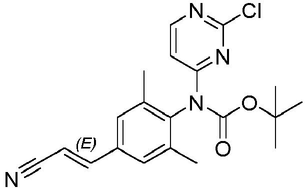

In a round bottomed flask, equipped with a mechanical stirrer, a water condenser, a temperature controller, under inert gas, sodium *tert*-amylate (58 g) and dimethylsulfoxide (250 mL) are added and stirred until a clear solution is obtained. A solution containing *tert-*butyl {4-[(E)-2-cyanoethenyl]-2,6-dimethylphenyl } carbamate (118 g) in dimethylsulfoxide (200 mL) is added. The resulting mixture is stirred at room temperature for 1 h. After this period of time, it is transferred to a solution of 2,4-dichloropyrimidine (86 g) in dimethylsulfoxide (200 mL). The resulting mixture is kept under stirring for 1 h before quenching on a mixture of water and *n*-butanol at 60°C. The resulting solid was collected by filtration and dried to obtain *tert*-butyl (2-chloropyrimidin-4-yl){4-[(E)-2-cyanoethenyl]-2,6-dimethylphenyl}carbamate (147 g, 80% yield). Purity of the product was found to be 95.3 % as assayed by HPLC.

Crude *tert*-butyl (2-chloropyrimidin-4-yl){4-[(E)-2-cyanoethenyl]-2,6-dimethylphenyl}carbamate (52 g) was dissolved in *n*-butanol, the mixture is heated to reflux and maintained until a clear solution is obtained. After cooling, the solid is filtered and dried to afford *tert*-butyl (2-chloropyrimidin-4-yl){4-[(E)-2-cyanoethenyl]-2,6-dimethylphenyl}carbamate (45 g, 87% yield). Purity of the product was found to be 99.0 % as assayed by HPLC. m.p. (DSC) 143 °C.

¹H NMR (300 MHz, DMSO): δ (ppm) 8.64 (d, *J* = 5.9 Hz, 1H), 8.09 (d, *J* = 5.9 Hz, 1H), 7.61 (d, *J* = 16.7 Hz, 1H), 7.48 (s, 2H), 6.47 (d, *J* = 16.7 Hz, 1H), 2.02 (s, 6H), 1.35 (s, 9H).

¹³C NMR (75 MHz, DMSO): δ (ppm) 160.8, 160.5, 159.0, 151.1, 149.9, 139.3, 136.1 (2C), 133.3, 127.6 (2C), 118.8, 110.5, 97.3, 83.3, 27.3 (3C), 17.3 (2C).

### Synthesis of 4- [(4- {4- [(E)-2-cyanoethenyl] -2,6-dimethylanilino} pyrimidin-2-yl)amino]benzonitrile hydrochloride (Rilpivirine hydrochloride).

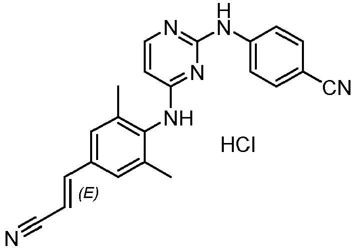

In a round bottomed flask, equipped with a mechanical stirrer, a water condenser, a temperature controller, under inert gas, 4-aminobenzonitrile (4.6 g), glacial acetic acid (7.5 mL), deionized water (7.5 mL) are heated to reflux. A 34% by weight aqueous solution of hydrochloric acid (1.4 g) is added. *Tert*-butyl (2-chloropyrimidin-4-yl){4-[(E)-2-cyanoethenyl]-2,6-dimethylphenyl}carbamate (5.0 g) is added as a solution in glacial acetic acid (15 mL). The mixture is refluxed for 1 h. After cooling, the solid is filtered and dried to give 4-[(4- f 4-[(E)-2-cyanoethenyl]-2,6-dimethylanilino}pyrimidin-2-yl)amino]benzonitrile hydrochloride (3.7 g, 71% yield). Purity of the product was found to be 93.9 % by LCAP.

4-[(4-{4-[(E)-2-cyanoethenyl]-2,6-dimethylanilino}pyrimidin-2-yl)amino]benzonitrile hydrochloride (3.5 g) was suspended in glacial acetic acid (21 mL) and deionized water (21 mL), the mixture is refluxed until a clear solution is obtained. After cooling, the solid is filtered and dried to afford purified 4-[(4-{4-[(E)-2-cyanoethenyl]-2,6-dimethylanilino}pyrimidin-2-yl)amino]benzonitrile hydrochloride (2.9 g, 83% yield). Purity of the product was found to be 99.7 % by LCAP.

The identity of the product was confirmed by mass spectrometry, comparison with an authentic sample by HPLC and comparisons of experimental ¹H NMR spectrum and XRPD pattern with literature data (US 7,956,063 B2). The product was unambiguously identified as the A form (anhydrous hydrochloride) of rilpivirine as shown on Figure 1.

m.p. > 290°C dec. (DSC).

¹H NMR (300 MHz, DMSO): δ (ppm) 11.27 (s, 1H), 10.92 (s, 1H), 8.12 (d, *J* = 6.9 Hz, 1H), 8.02 - 7.80 (m, 1H), 7.69 (d, *J* = 16.7 Hz, 1H), 7.54 (s, 2H), 7.42 (dd, *J* = 22.7, 8.1 Hz, 3H), 6.67 (d, *J* = 6.9 Hz, 1H), 6.53 (d, *J* = 16.7 Hz, 1H), 2.18 (s, 6H).

### Example 2

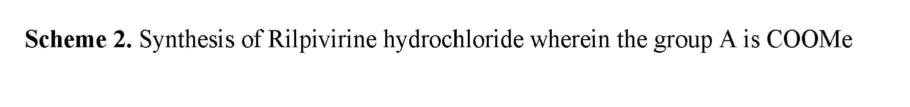

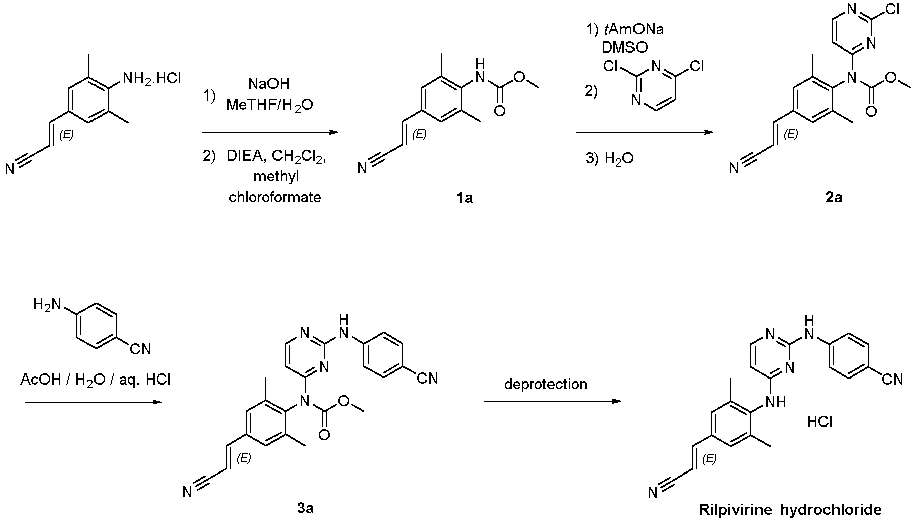

### Synthesis of methyl {4-[(E)-2-cyanoethenyl]-2,6-dimethylphenyl}carbamate 1a.

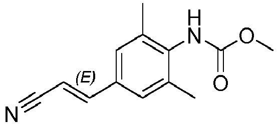

In a round bottom flask under nitrogen were mixed (*E*)-3-(4amino-3,5-dimethylphenyl)acrylonitrile (1 g, 6 mmol, 1 equiv) and *N*,*N*-diisopropylethylamine (1.7 mL, 9.6 mmol, 1.6 equiv) in dry dichloromethane (20 mL). The mixture was cooled to 0°C before the dropwise addition of methyl chloroformate (0.7 mL, 9.0 mmol, 1.5 equiv). The reaction was allowed to warm up to room temperature and stirred for 72 h. The resulting mixture was then washed with a 1 M aqueous solution of hydrochloric acid and water before drying the organic layer over anhydrous sodium sulfate and concentrating down. Recrystallization of the crude solid in hot ethyl acetate gave the desired compound as a light brown solid (860 mg, 62%).

¹H NMR (400 MHz, CDCl₃) δ (ppm) = 7.31 (dt, *J* = 16.6, 0.6 Hz, 1H), 7.18 - 7.12 (m, 2H), 5.82 (d, *J* = 16.6 Hz, 1H), 3.76 (br. s, 3H), 2.30 - 2.26 (m, 6H).

¹³C NMR (101 MHz, CDCl₃) δ (ppm) = 150.2, 136.6, 132.3, 127.4, 118.3, 96.4, 52.8, 18.6.

MS (ESI): m/z = 229.0 (M-H)⁻

### Synthesis of methyl (2-chloropyrimidin-4-yl){4-[(E)-2-cyanoethenyl]-2,6-dimethylphenyl}carbamate 2a.

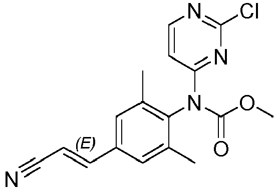

In a reaction vial under nitrogen, was dissolved sodium tert-amylate (462 mg, 4.2 mmol, 1.2 equiv) in dry dimethylsulfoxide (2 mL). The resulting mixture was stirred for 1 h until a clear solution was obtained before the dropwise addition of a solution of methyl (E)-(4-(2-cyanoethenyl)-2,6-dimethylphenyl)carbamate **1a** (800 mg, 3.5 mmol, 1.0 equiv) in dry dimethylsulfoxide (2 mL). The stirring was continued for 1 h before its dropwise transfer to a solution of 2,4-dichloropyrimidine (678 mg, 4.6 mmol, 1.3 equiv) in dry dimethylsulfoxide (1.5 mL). After 1 h, water is added, the desired product was collected by filtration and dried under vacuum to afford a pale brown solid (1.0 g, 84%).

¹H NMR (400 MHz, CDCl₃) δ (ppm) = 8.51 (d, *J* = 5.9 Hz, 1H), 8.15 (d, *J* = 5.9 Hz, 1H), 7.36 (dt, *J* = 16.6, 0.5 Hz, 1H), 7.22 (h, *J* = 0.6 Hz, 2H), 5.89 (d, *J* = 16.6 Hz, 1H), 3.78 (s, 3H), 2.10 - 2.04 (m, 6H).

¹³C NMR (101 MHz, CDCl₃) δ (ppm) = 161.1, 160.5, 160.0, 153.9, 150.0, 139.2, 137.1, 133.6, 127.6, 118.2, 110.0, 97.1, 54.5, 18.1.

MS (ESI): m/z = 343.1 (M+H)⁺

### Synthesis of methyl (E)-(2-((4-cyanophenyl)amino)pyrimidin-4-yl)(4-(2-cyanoethenyl)-2,6-dimethylphenyl)carbamate 3a

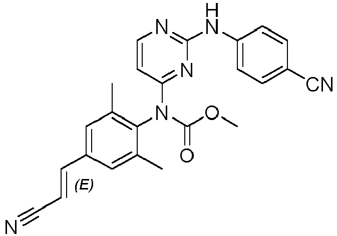

In a reaction vial were heated to reflux a mixture of 4-aminobenzonitrile (886 mg, 7.5 mmol, 3.0 equiv) in a 1:1 mixture of acetic acid and deionized water (3 mL). 37% hydrochloric acid in water was added (240 µL, 2.9 mmol, 1.2 equiv) before the introduction of a solution of methyl (*E*)-(2-chloropyrimidin-4-yl)(4-(2-cyanovinyl)-2,6-dimethylphenyl)carbamate **2a** (857 mg, 2.5 mmol, 1.0 equiv) in acetic acid (9 mL). The reflux was kept for one hour before cooling down the resulting mixture. Water was added and the desired product precipitated. The crude solid was then dissolved in ethyl acetate and washed with a saturated solution of sodium hydrogen carbonate, followed by brine. Organics were dried over anhydrous sodium sulfate and concentrated down to give the desired product (780 mg, 73%).

¹H NMR (400 MHz, CDCl₃) δ (ppm) = 8.40 (d, *J* = 5.8 Hz, 1H), 7.79 (d, *J* = 5.8 Hz, 1H), 7.47 (d, *J* = 16.6 Hz, 1H), 7.41 (br. s, 1H), 7.31 (s, 2H), 7.23 - 7.18 (m, 2H), 7.07 - 7.01 (m, 2H), 5.98 (d, *J* = 16.6 Hz, 1H), 3.78 (s, 3H), 2.11 (s, 6H).

¹³C NMR (101 MHz, CDCl₃) δ (ppm) = 160.1, 159.4, 158.5, 153.9, 149.6, 143.4, 140.5, 137.6, 133.5, 132.9, 127.5, 119.3, 117.9, 117.7, 104.4, 103.9, 97.6, 54.3, 18.1.

MS (ESI): m/z = 425.1 (M+H)⁺

### Synthesis of 4-[(4-{4-[(E)-2-cyanoethenyl]-2,6-dimethylanilino}pyrimidin-2-yl)amino]benzonitrile hydrochloride (Rilpivirine hydrochloride).

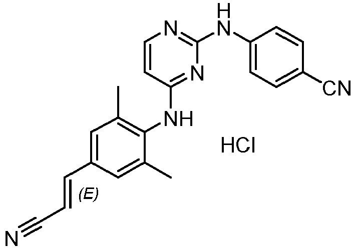

In a microwave tube were mixed methyl (*E*)-(2-((4-cyanophenyl)amino)pyrimidin-4-yl)(4-(2-cyanoethenyl)-2,6-dimethylphenyl)carbamate **3a** (127 mg, 0.3 mmol, 1 equiv) and lithium chloride (127 mg, 3 mmol, 10 equiv) in acetic acid (1.5 mL). The reaction was heated by microwave irradiation during 3 cycles of 20 min at 150°C. Water was then added and the mixture was extracted with ethyl acetate. Organics were washed with a saturated aqueous solution of sodium hydrogen carbonate before drying over anhydrous sodium sulfate and concentration. The crude mixture was purified by flash chromatography (SiO₂, petroleum ether / ethyl acetate 6:1 to 1:1) to give 64 mg of Rilpivirine free base (40%) 44 mg of the free base (0.12 mmol) was dissolved in boiling isopropanol (5 mL) and 37% aqueous hydrochloric acid was added (10 µL, 0.12 mmol, 1 equiv). The mixture was cooled down, and the desired Rilpivirine hydrochloride was collected by filtration (38.5 mg, 80%).

¹H, ¹³C NMR and MS matched the analytical reference.

### Example 3

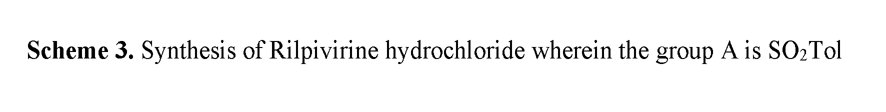

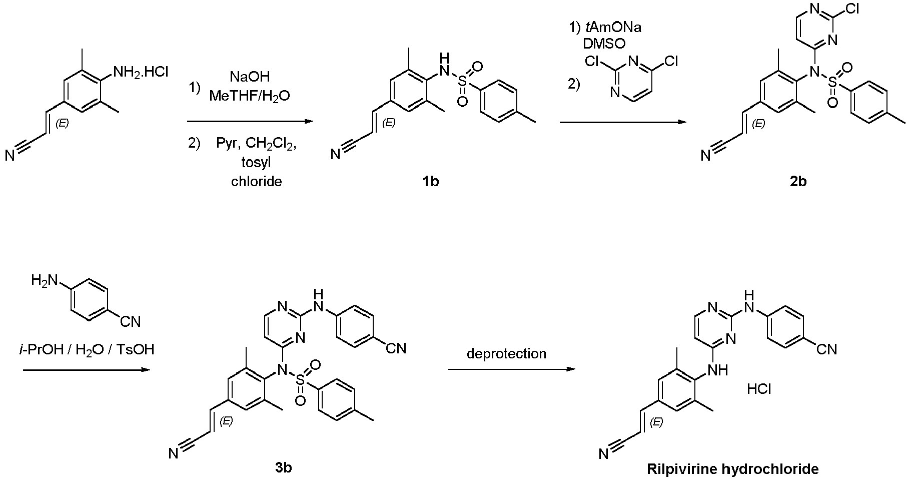

### Synthesis of (E)-N-(4-(2-cyanoethenyl)-2,6-dimethylphenyl)-4-methylbenzene-sulfonamide 1b

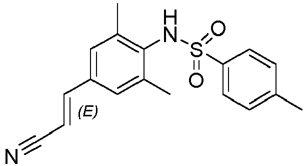

In a round bottom flask under nitrogen were mixed (E)-3-(4amino-3,5-dimethylphenyl)acrylonitrile (1.0 g, 6.0 mmol, 1.0 equiv) and pyridine (956 µL, 12 mmol, 2.0 equiv) in dry dichloromethane (20 mL). The mixture was cooled to 0°C before the portionwise addition of tosyl chloride (2.3 g, 12 mmol, 2.0 equiv). The reaction was allowed to warm up to room temperature and stirred for 72 h. The mixture was then washed with a 1 M aqueous solution of hydrochloric acid, water, a saturated aqueous solution of sodium hydrogen carbonate and finally water before drying the organic layer over anhydrous sodium sulfate and concentration. The crude solid was recrystallized from a mixture of petroleum ether and ethyl acetate to give the desired compound as a brown solid (1.25 g, 63%).

¹H NMR (400 MHz, CDCl₃) δ (ppm) = 7.59 - 7.55 (m, 2H), 7.27 - 7.21 (m, 3H), 7.06 (br. s, 2H), 6.47 (br. s, 1H), 2.40 (s, 3H), 2.03 (s, 6H).

¹³C NMR (101 MHz, CDCl₃) δ (ppm) = 149.9, 144.1, 138.7, 137.6, 135.5, 132.6, 129.9, 127.8, 127.2, 118.2, 96.8, 21.7, 18.9.

MS (ESI) m/z = 325.0 (M-H)⁻

### Synthesis of (E)-N-(2-chloropyrimidin-4-yl)-N-(4-(2-cyanoethenyl)-2,6-dimethylphenyl)-4-methylbenzenesulfonamide 2b

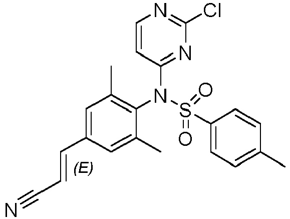

In a reaction vial under nitrogen, was dissolved sodium tert-amylate (396 mg, 3.6 mmol, 1.2 equiv) in dry dimethylsulfoxide (1.6 mL). The resulting mixture was stirred for 1 h until a clear solution was obtained before the dropwise addition of a solution of (*E*)-N-(4-(2-cyanoethenyl)-2,6-dimethylphenyl)-4-methylbenzenesulfonamide **2a** (986 mg, 3.0 mmol, 1.0 equiv) in dry dimethylsulfoxide (1.6 mL). The stirring was continued for 1 h before its dropwise transfer to a solution of 2,4-dichloropyrimidine (582 mg, 3.9 mmol, 1.3 equiv) in dry dimethylsulfoxide (1.5 mL). After 96 h, the mixture was filtered and the resulting solid washed with dimethylsulfoxide to give title compound as a beige solid (840 mg, 80%).

¹H NMR (400 MHz, CDCl₃) δ (ppm) = 8.22 - 8.16 (m, 3H), 7.40 - 7.34 (m, 3H), 7.31 (br. s, 2H), 5.97 - 5.91 (m, 2H), 2.47 (s, 3H), 2.17 (s, 6H).

¹³C NMR (101 MHz, CDCl₃) δ (ppm) = 160.3, 160.2, 159.7, 149.3, 145.7, 140.0, 136.8, 136.1, 134.9, 130.9, 129.3, 128.4, 117.9, 105.4, 98.5, 21.9, 19.0.

MS (ESI) m/z = 439.1 (M+H)

### Synthesis of (E)-N-(2-((4-cyanophenyl)amino)pyrimidin-4-yl)-N-(4-(2-cyanoethenyl)-2,6-dimethylphenyl)-4-methylbenzenesulfonamide 3b

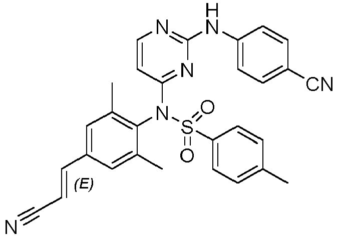

In a reaction vial were heated to reflux a mixture of 4-aminobenzonitrile (850 mg, 7.2 mmol, 3 equiv) in a 1:1 mixture of isopropanol and deionized water (3.2 mL). *p-*toluenesulfonic acid monohydrate was added (500 mg, 2.6 mmol, 1.1 equiv) before the introduction of a solution of (*E*)-N-(2-chloropyrimidin-4-yl)-N-(4-(2-cyanoethenyl)-2,6-dimethylphenyl)-4methylbenzenesulfonamide **2b** (840 mg, 2.4 mmol, 1 equiv) in isopropanol (9 mL). The reflux was kept for 7 h before cooling down the resulting mixture. Water was added and the desired product was extracted with ethyl acetate and washed with a saturated solution of sodium hydrogen carbonate, followed by brine. Organics were dried over anhydrous sodium sulfate and concentrated down. Trituration in acetone of the resulting crude solid yielded the desired compound (562 mg, 45%).

¹H NMR (400 MHz, CDCl₃) δ (ppm) = 8.17 (d, *J* = 5.8 Hz, 1H), 7.99 - 7.94 (m, 2H), 7.58 - 7.54 (m, 2H), 7.51 - 7.47 (m, 2H), 7.44 - 7.37 (m, 2H), 7.27 (d, *J* = 8.8 Hz, 4H), 6.19 (d, *J* = 5.8 Hz, 1H), 5.95 (d, *J* = 16.6 Hz, 1H), 2.43 (s, 3H), 2.10 (s, 7H).

¹³C NMR (101 MHz, CDCl₃) δ (ppm) = 159.8, 159.0, 158.5, 149.4, 145.5, 143.3, 140.3, 138.0, 136.7, 134.4, 133.2, 129.7, 129.2, 128.1, 119.4, 118.9, 117.9, 105.2, 100.7, 98.2, 21.8, 19.1.

MS (ESI) m/z = 521.1 (M+H)⁺

### Synthesis of 4-[(4-14-[(E)-2-cyanoethenyl]-2,6-dimethylanilinolpyrimidin-2-yl)amino]benzonitrile hydrochloride (Rilpivirine hydrochloride).

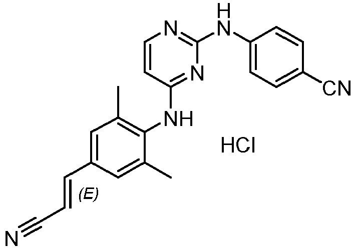

In a reaction vial were mixed methyl (*E*)-N-(2-((4-cyanophenyl)amino)pyrimidin-4-yl)-N-(4-(2-cyanoethenyl)-2,6-dimethylphenyl)-4-methylbenzenesulfonamide **3b** (156 mg, 0.3 mmol, 1 equiv) and zinc dust (106 mg, 3 mmol, 10 equiv) in acetic acid (3 mL). The reaction mixture was heated at 100°C for 1 h. TLC showed incomplete conversion, thus zinc dust (106 mg, 3 mmol, 10 equiv) was added and the mixture was stirred at 100°C for another 30 min. Water was then added and the mixture was extracted with ethyl acetate. Organics were washed with a saturated aqueous solution of sodium hydrogen carbonate before being dried over anhydrous sodium sulfate and evaporated. The crude mixture was then dissolved in boiling isopropanol (9 mL) and 37% aqueous hydrochloric acid was added (25 µL, 0.3 mmol, 1 equiv). The mixture was cooled down, and the desired Rilpivirine hydrochloride was collected by filtration (61 mg, 50%).

¹H, ¹³C NMR and MS matched the analytical reference.

### Example 4

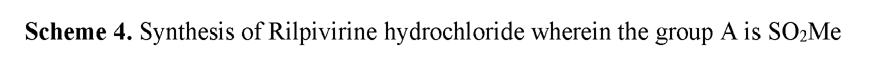

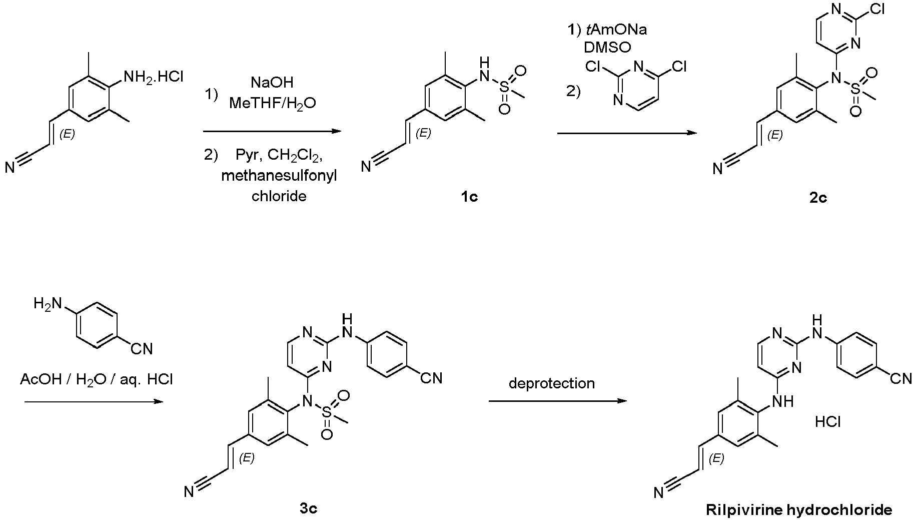

### Synthesis of (E)-N-(4-(2-cyanoethenyl)-2,6-dimethylphenyl)methanesulfonamide 1c

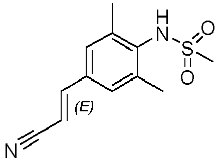

In a round bottom flask under nitrogen were mixed (E)-3-(4-amino-3,5-dimethylphenyl)acrylonitrile (1.0 g, 6.0 mmol, 1.0 equiv) and pyridine (956 µL, 12 mmol, 2.0 equiv) in dry dichloromethane (20 mL). The mixture was cooled to 0°C before the dropwise addition of methanesulfonyl chloride (927 µL, 12 mmol, 2.0 equiv). The reaction was allowed to warm up to room temperature and stirred for 48 h. The mixture was then washed with a 1 M aqueous solution of hydrochloric acid, water, a saturated aqueous solution of sodium hydrogen carbonate and finally water before drying the organic layer over anhydrous sodium sulfate and concentration. The crude solid was recrystallized from a mixture of petroleum ether and ethyl acetate to give the desired compound as a brown solid (895 mg, 60%).

¹H NMR (400 MHz, CDCl₃) δ (ppm) = 7.31 (d, *J* = 16.6 Hz, 1H), 7.20 (br. s, 2H), 5.99 (br. s, 1H), 5.85 (d, *J* = 16.6 Hz, 1H), 3.13 (s, 3H), 2.44 (s, 6H).

¹³C NMR (101 MHz, CDCl₃) δ (ppm) = 149.7, 138.2, 135.7, 133.0, 127.9, 118.1, 97.2, 42.4, 19.5.

MS (ESI) m/z = 249.0 (M-H)⁻

### Synthesis of (E)-N-(2-chloropyrimidin-4-yl)-N-(4-(2-cyanoethenyl)-2,6-dimethylphenyl)methanesulfonamide 2c

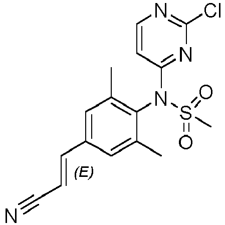

In a reaction vial under nitrogen, was dissolved sodium tert-amylate (36 mg, 3.6 mmol, 1.2 equiv) in dry dimethylsulfoxide (1.6 mL). The resulting mixture was stirred for 1 h until a clear solution was obtained before the dropwise addition of a solution (E)-N-(4-(2-cyanoethenyl)-2,6-dimethylphenyl)methanesulfonamide **2b** (750 mg, 3 mmol, 1 equiv) in dry dimethylsulfoxide (1.6 mL). The stirring was continued for 1 h before its dropwise transfer to a solution of 2,4-dichloropyrimidine (582 mg, 3.9 mmol, 1.3 equiv) in dry dimethylsulfoxide (1.5 mL). After 1 h, the mixture was diluted with brine and extracted with ethyl acetate. Organics were dried over anhydrous sodium sulfate and concentrated down. The desired compound was obtained after recrystallization of the crude material from a mixture of petroleum ether and ethyl acetate (710 mg, 65%).

¹H NMR (400 MHz, CDCl₃) δ (ppm) = 8.27 (d, *J* = 5.8 Hz, 1H), 7.35 (d, *J* = 16.7 Hz, 1H), 7.32 - 7.29 (m, 2H), 5.98 (d, *J* = 5.7 Hz, 1H), 5.92 (d, *J* = 16.6 Hz, 1H), 3.75 (s, 3H), 2.26 (s, 6H).

¹³C NMR (101 MHz, CDCl₃) δ (ppm) = 160.9, 160.4, 160.2, 149.1, 139.6, 136.0, 135.0, 128.4, 117.7, 105.3, 98.6, 44.6, 18.9.

MS (ESI) m/z = 363.0 (M+H)⁺

### Synthesis of (E)-N-(2-((4-cyanophenyl)amino)pyrimidin-4-yl)-N-(4-(2-cyanoethenyl)-2,6-dimethylphenyl)methanesulfonamide 3c

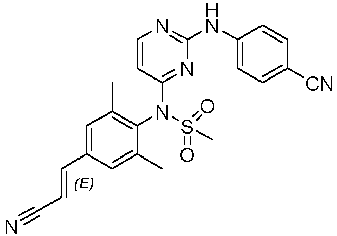

In a reaction vial were heated to reflux a mixture of 4-aminobenzonitrile (588 mg, 5.0 mmol, 3 equiv) in a 1:1 mixture of acetic acid and deionized water (2 mL). 37% hydrochloric acid in water was added (160 µL, 2.0 mmol, 1.2 equiv) before the introduction of a solution of (*E*)-N-(2-chloropyrimidin-4-yl)-N-(4-(2-cyanoethenyl)-2,6-dimethylphenyl)methanesulfonamide 2c (600 mg, 1.7 mmol, 1 equiv) in acetic acid (6 mL). The reflux was kept for one hour before cooling down the resulting mixture. Water was added and the desired product precipitated. The crude solid was then dissolved in ethyl acetate and washed with a saturated solution of sodium hydrogen carbonate, followed by brine. Organics were dried over anhydrous sodium sulfate and concentrated down to give the desired product (570 mg, 77%).

¹H NMR (400 MHz, CDCl₃) δ (ppm) = 8.17 (d, *J* = 5.7 Hz, 1H), 7.88 (br. s, 1H), 7.83 - 7.78 (m, 2H), 7.65 - 7.59 (m, 2H), 7.37 (d, *J* = 16.7 Hz, 1H), 7.30 (s, 2H), 5.93 (d, *J* = 16.6 Hz, 1H), 5.65 (d, *J* = 5.7 Hz, 1H), 3.63 (s, 3H), 2.29 (s, 6H).

¹³C NMR (101 MHz, CDCl₃) δ (ppm) = 160.0, 159.5, 158.4, 149.3, 143.2, 139.8, 136.7, 134.8, 133.4, 128.3, 119.3, 119.3, 117.9, 105.5, 99.4, 98.4, 44.0, 18.9.

MS (ESI) m/z = 445.1 (M+H)⁺

### Synthesis of 4-[(4-14-[(E)-2-cyanoethenyl]-2,6-dimethylanilinolpyrimidin-2-yl)amino]benzonitrile hydrochloride (Rilpivirine hydrochloride).

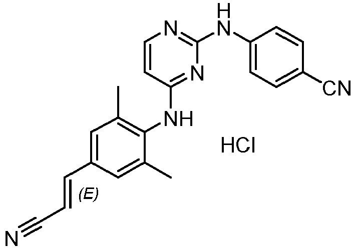

In a round bottom flask were mixed (*E*)-N-(2-((4-cyanophenyl)amino)pyrimidin-4-yl)-N-(4-(2-cyanoethenyl)-2,6-dimethylphenyl)methanesulfonamide **3c** (133 mg, 0.3 mmol, 1 equiv) and potassium carbonate (330 mg, 2.4 mmol, 8 equiv) in DMF (3 mL). The reaction mixture was heated at 80°C for 48 h. Water was then added and the mixture was extracted with ethyl acetate. Organics were washed with brine before being dried over anhydrous sodium sulfate and evaporated. The crude mixture was then dissolved in boiling isopropanol (12 mL) and 37% aqueous hydrochloric acid was added (25 µL, 0.3 mmol, 1 equiv). The mixture was cooled down, and the desired Rilpivirine hydrochloride was collected by filtration (71 mg, 59%).

¹H, ¹³C NMR and MS matched the analytical reference.

## Claims

1. Process for the preparation of Rilpivirine or a pharmaceutically acceptable salt or solvate thereof comprising the following steps in the following order:
a) preparing a compound of formula II: 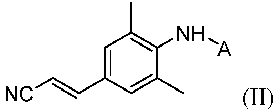 wherein **A** is an amine protecting group selected from alkyloxycarbonyl, arylalkyloxycarbonyl, alkylcarbonyl, arylsulfonyl, alkylsulfonyl, haloalkylsulfonyl, alkyl, alkenyl and arylalkyl,
b) reacting said compound of formula II with a 2,4-dihalogenopyrimidine in the presence of a base to obtain compound of formula III: 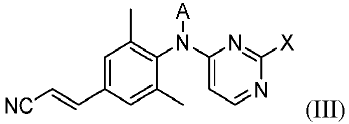 wherein **A** is an amine protecting group selected from alkyloxycarbonyl, arylalkyloxycarbonyl, alkylcarbonyl, arylsulfonyl, alkylsulfonyl, haloalkylsulfonyl, alkyl, alkenyl and arylalkyl and **X** is a halogen,
c) reacting said compound of formula III with 4-aminobenzonitrile in the presence of an acid, and
d) cleaving the group **A**,
wherein steps a), b, and c) are carried out successively in this order and step d) is carried out concomitantly with step c) or after step c).

2. Process according to claim 1, wherein the amine protecting group is selected from alkyloxycarbonyl, arylsulfonyl and alkylsulfonyl.

3. Process according to any of claims 1 and 2, wherein the base used in step b) is selected from alkali metal salts of alcohols and amides.

4. Process according to any of claims 1 to 3, wherein the solvent used in step b) is selected from polar aprotic solvents and aromatic non-polar solvents.

5. Process according to any of claims 1 to 4, wherein step b) is run at a temperature ranging from 10°C to 60°C.

6. Process according to any of claims 1 to 5, wherein the acid used in step c) is selected from acetic acid, hydrochloric acid, *p*-toluenesulfonic acid, methanesulfonic acid, formic acid, trifluoroacetic acid, any supported acidic catalyst and mixtures thereof.

7. Process according to any of claims 1 to 6, wherein the step c) is performed in a solvent selected from polar solvents and aromatic non-polar solvents, optionally as a mixture with water.

8. Process according to any of claims 1 to 7, wherein step c) is performed at a temperature ranging from 50°C to 200°C.

9. Process according to claim 1 resulting in polymorphic form A of Rilpirivine hydrochloride, wherein the compound of formula III is reacted in step c) with 4-aminobenzonitrile in the presence of hydrochloric acid and wherein step d) is carried out concomitantly with step c) using a mixture of glacial acetic acid and water as solvent.

10. Compound of formula II: 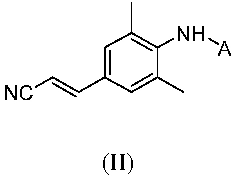 and suitable salts thereof,
wherein **A** is an amine protecting group selected from alkyloxycarbonyl, alkylcarbonyl, arylsulfonyl, alkylsulfonyl, haloalkylsulfonyl, alkyl, alkenyl and arylalkyl.

11. Compound according to claim 10, wherein the amine protecting group is selected from alkyloxycarbonyl, arylsulfonyl and alkylsulfonyl.

12. Compound of formula III: 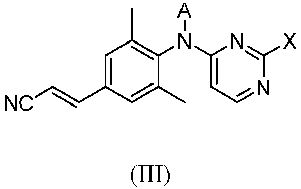 and suitable salts thereof,
wherein **A** is an amine protecting group selected from alkyloxycarbonyl, arylalkyloxycarbonyl, alkylcarbonyl, arylsulfonyl, alkylsulfonyl, haloalkylsulfonyl, alkyl, alkenyl and arylalkyl and **X** is a halogen.

13. Compound according to claim 12, wherein the amine protecting group is selected from alkyloxycarbonyl, arylsulfonyl and alkylsulfonyl.

14. Use of compound according to any of claims 10 and 11 as a synthetic intermediate for the preparation of Rilpivirine or a pharmaceutically acceptable salt or solvate thereof.

15. Use of compound according to any of claims 12 and 13 as a synthetic intermediate for the preparation of Rilpivirine or a pharmaceutically acceptable salt or solvate thereof.

## Patentansprüche

1. Verfahren zur Herstellung von Rilpivirin oder eines pharmazeutisch akzeptablen Salzes oder Solvats davon, das die folgenden Schritte in der folgenden Reihenfolge umfasst:
a) Herstellen einer Verbindung der Formel II: 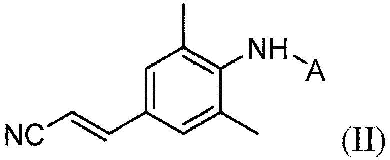 wobei **A** eine Aminschutzgruppe ausgewählt aus Alkyloxycarbonyl, Arylalkyloxycarbonyl, Alkylcarbonyl, Arylsulfonyl, Alkylsulfonyl, Halogenalkylsulfonyl, Alkyl, Alkenyl und Arylalkyl ist,
b) Umsetzen der Verbindung der Formel II mit einem 2,4-Dihalogenpyrimidin in der Gegenwart einer Base, um eine Verbindung der Formel III zu erhalten: 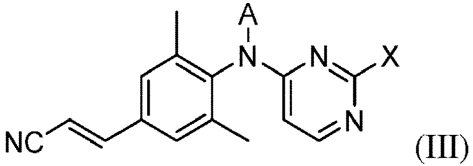 wobei **A** eine Aminschutzgruppe ausgewählt aus Alkyloxycarbonyl, Arylalkyloxycarbonyl, Alkylcarbonyl, Arylsulfonyl, Alkylsulfonyl, Halogenalkylsulfonyl, Alkyl, Alkenyl und Arylalkyl ist, und **X** ein Halogen ist,
c) Umsetzen der Verbindung der Formel III mit 4-Aminobenzonitril in Gegenwart einer Säure, und
d) Abspalten der Gruppe A,
wobei Schritte a), b) und c) nacheinander in dieser Reihenfolge durchgeführt werden und Schritt d) gleichzeitig mit Schritt c) oder nach Schritt c) durchgeführt wird.

2. Verfahren nach Anspruch 1, wobei die Aminschutzgruppe ausgewählt ist aus Alkyloxycarbonyl, Arylsulfonyl und Alkylsulfonyl.

3. Verfahren nach einem der Ansprüche 1 bis 2, wobei die in Schritt b) verwendete Base ausgewählt ist aus Alkalimetallsalzen von Alkoholen und Amiden.

4. Verfahren nach einem der Ansprüche 1 bis 3, wobei das in Schritt b) verwendete Lösungsmittel ausgewählt ist aus polaren aprotischen Lösungsmitteln und aromatischen unpolaren Lösungsmitteln.

5. Verfahren nach einem der Ansprüche 1 bis 4, wobei Schritt b) bei einer Temperatur im Bereich von 10°C bis 60°C durchgeführt wird.

6. Verfahren nach einem der Ansprüche 1 bis 5, wobei die in Schritt c) verwendete Säure ausgewählt ist aus Essigsäure, Salzsäure, p-Toluolsulfonsäure, Methansulfonsäure, Ameisensäure, Trifluoressigsäure, jeglichem geeigneten sauren Katalysator und Mischungen davon.

7. Verfahren nach einem der Ansprüche 1 bis 6, wobei der Schritt c) in einem Lösungsmittel, ausgewählt aus polaren Lösungsmitteln und aromatischen unpolaren Lösungsmitteln, gegebenenfalls als eine Mischung mit Wasser, durchgeführt wird.

8. Verfahren nach einem der Ansprüche 1 bis 7, wobei Schritt c) bei einer Temperatur im Bereich von 50 °C bis 200 °C durchgeführt wird.

9. Verfahren nach Anspruch 1, das zur polymorphen Form A von Rilpirivin-Hydrochlorid führt, wobei die Verbindung der Formel III in Schritt c) mit 4-Aminobenzonitril in der Gegenwart von Salzsäure umgesetzt wird und wobei Schritt d) gleichzeitig mit Schritt c) unter Verwendung einer Mischung aus Eisessig und Wasser als Lösungsmittel durchgeführt wird.

10. Verbindung der Formel II: 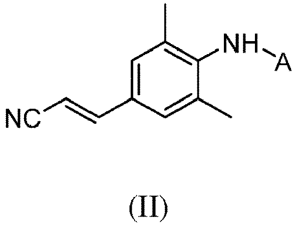 und geeignete Salze davon,
wobei **A** eine Aminschutzgruppe ausgewählt aus Alkyloxycarbonyl, Alkylcarbonyl, Arylsulfonyl, Alkylsulfonyl, Halogenalkylsulfonyl, Alkyl, Alkenyl und Arylalkyl ist.

11. Verbindung nach Anspruch 10, wobei die Aminschutzgruppe ausgewählt ist aus Alkyloxycarbonyl, Arylsulfonyl und Alkylsulfonyl.

12. Verbindung der Formel III: 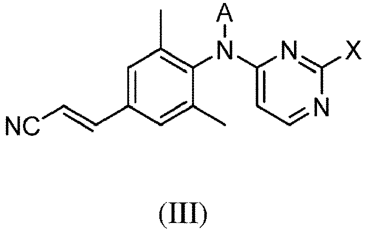 und geeignete Salze davon,
wobei **A** eine Aminschutzgruppe ausgewählt aus Alkyloxycarbonyl, Arylalkyloxycarbonyl, Alkylcarbonyl, Arylsulfonyl, Alkylsulfonyl, Halogenalkylsulfonyl, Alkyl, Alkenyl und Arylalkyl ist, und **X** ein Halogen ist.

13. Verbindung nach Anspruch 12, wobei die Aminschutzgruppe ausgewählt ist aus Alkyloxycarbonyl, Arylsulfonyl und Alkylsulfonyl.

14. Verwendung der Verbindung nach einem der Ansprüche 10 und 11 als synthetisches Zwischenprodukt für die Herstellung von Rilpivirin oder eines pharmazeutisch akzeptablen Salzes oder Solvats davon.

15. Verwendung der Verbindung nach einem der Ansprüche 12 und 13 als synthetisches Zwischenprodukt für die Herstellung von Rilpivirin oder eines pharmazeutisch akzeptablen Salzes oder Solvats davon.

## Revendications

1. Procédé de préparation de rilpivirine ou d'un sel ou solvate pharmaceutiquement acceptables de celle-ci comprenant les étapes suivantes dans l'ordre suivant :
a) préparation d'un composé de formule II : 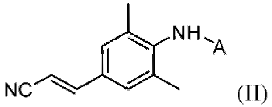 dans lequel A est un groupe protecteur amine sélectionné parmi un alkyloxycarbonyle, un arylalkyloxycarbonyle, un alkylcarbonyle, un arylsulfonyle, un alkylsulfonyle, un halogénoalkylsulfonyle, un alkyle, un alcényle et un arylalkyle,
b) mise en réaction dudit composé de formule II avec une 2,4-dihalogénopyrimidine en présence d'une base pour obtenir un composé de formule III : 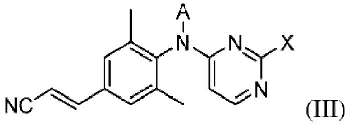 dans laquelle A est un groupe protecteur amine sélectionné parmi un alkyloxycarbonyle, un arylalkyloxycarbonyle, un alkylcarbonyle, un arylsulfonyle, un alkylsulfonyle, un halogénoalkylsulfonyle, un alkyle, un alcényle et un arylalkyle et X est un halogène,
c) mise en réaction dudit composé de formule III avec un 4-aminobenzonitrile en présence d'un acide, et
d) clivage du groupe A,
dans lequel les étapes a), b), et c) sont réalisées successivement dans cet ordre et l'étape d) est réalisée en même temps que l'étape c) ou après l'étape c).

2. Procédé selon la revendication 1, dans lequel le groupe protecteur amine est sélectionné parmi un alkyloxycarbonyle, un arylsulfonyle et un alkylsulfonyle.

3. Procédé selon l'une quelconque des revendications 1 et 2, dans lequel la base utilisée à l'étape b) est sélectionnée parmi des sels de métaux alcalins d'alcools et d'amides.

4. Procédé selon l'une quelconque des revendications 1 à 3, dans lequel le solvant utilisé à l'étape b) est sélectionné parmi des solvants aprotiques polaires et des solvants aromatiques non polaires.

5. Procédé selon l'une quelconque des revendications 1 à 4, dans lequel l'étape b) est exécutée à une température dans la plage de 10 °C à 60 °C.

6. Procédé selon l'une quelconque des revendications 1 à 5, dans lequel l'acide utilisé à l'étape c) est sélectionné parmi l'acide acétique, l'acide chlorhydrique, l'acide p-toluènesulfonique, l'acide méthanesulfonique, l'acide formique, l'acide trifluoroacétique, tout catalyseur acide supporté et des mélanges de ceux-ci.

7. Procédé selon l'une quelconque des revendications 1 à 6, dans lequel l'étape c) est réalisée dans un solvant sélectionné parmi des solvants polaires et des solvants aromatiques non polaires, facultativement sous la forme d'un mélange avec de l'eau.

8. Procédé selon l'une quelconque des revendications 1 à 7, dans lequel l'étape c) est réalisée à une température dans la plage de 50 °C à 200 °C.

9. Procédé selon la revendication 1 résultant en une forme polymorphe A de chlorhydrate de rilpivirine, dans lequel le composé de formule III est mis en réaction à l'étape c) avec un 4-aminobenzonitrile en présence d'acide chlorhydrique et dans lequel l'étape d) est réalisée en même temps que l'étape c) à l'aide d'un mélange d'acide acétique glacial et d'eau en tant que solvant.

10. Composé de formule II : 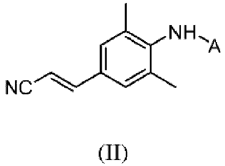 et les sels adaptés de celui-ci,
dans lequel A est un groupe protecteur amine sélectionné parmi un alkyloxycarbonyle, un alkylcarbonyle, un arylsulfonyle, un alkylsulfonyle, un halogénoalkylsulfonyle, un alkyle, un alcényle et un arylalkyle.

11. Composé selon la revendication 10, dans lequel le groupe protecteur amine est sélectionné parmi un alkyloxycarbonyle, un arylsulfonyle, un alkylsulfonyle.

12. Composé de formule III : 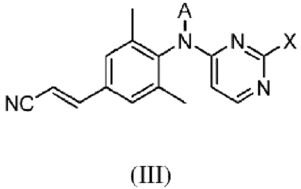 et les sels adaptés de celui-ci,
dans lequel A est un groupe protecteur amine sélectionné parmi un alkyloxycarbonyle, un arylalkyloxycarbonyle, un alkylcarbonyle, un arylsulfonyle, un alkylsulfonyle, un halogénoalkylsulfonyle, un alkyle, un alcényle et un arylalkyle et X est un atome d'halogène.

13. Composé selon la revendication 12, dans lequel le groupe protecteur amine est sélectionné parmi un alkyloxycarbonyle, un arylsulfonyle et un alkylsulfonyle.

14. Utilisation d'un composé selon l'une quelconque des revendications 10 et 11 en tant qu'intermédiaire de synthèse pour la préparation de rilpivirine ou d'un sel ou solvate pharmaceutiquement acceptables de celle-ci.

15. Utilisation d'un composé selon l'une quelconque des revendications 12 et 13 en tant qu'intermédiaire de synthèse pour la préparation de rilpivirine ou d'un sel ou solvate pharmaceutiquement acceptables de celle-ci.
